# EUROPÄISCHE PATENTANMELDUNG

(11) **EP 1 857 043 A2**
(43) Veröffentlichungstag der Anmeldung: **21.11.2007**
(21) Anmeldenummer: 07009864.5
(22) Anmeldetag: 16.05.2007
(51) Int. Cl.: A61B 3/135, A61B 3/14, A61B 3/107, A61B 3/10

(54) **Vorrichtung zur berührungslosen Untersuchung eines Auges**

(30) Priorität: 18.05.2006 DE 202006008017 U; 24.05.2006 DE 102006024473
(71) Anmelder: Rhine-tec Gesellschaft für Virtuelle Instrumentierung mbH, 47807 Krefeld (DE)
(72) Erfinder: Dahmen, Norbert, 47918 Tönisvorst (DE); Langstrof, Georg, 42781 Haan (DE)
(74) Vertreter: Eisenführ, Speiser & Partner

(57) **Zusammenfassung**

Vorrichtung zur berührungslosen Untersuchung eines Auges, enthaltend eine mit einer Spaltlampe 1 mechanisch gekoppelte Bildaufnahmeeinrichtung 2, mit welcher von einer Beleuchtungseinrichtung 3 der Spaltlampe 1 beleuchtete Bestandteile der Hornhaut und/oder ein Tränenfilm auf der Hornhautoberfläche erfassbar sind, und eine an die Bildaufnahmeeinrichtung 1 angeschlossene Bildwiedergabeeinrichtung 6, mit welcher die erfassten Bestandteile der Hornhaut und/oder der Tränenfilm im von der Spaltlampe erzeugten Schnittbild sichtbar darstellbar sind oder ist.

## Beschreibung

Die Erfindung betrifft eine Vorrichtung zur berührungslosen Untersuchung eines Auges, insbesondere eines menschlichen Auges.

Zur präoperativen Diagnostik sowie zur postoperativen Betreuung von Patienten, beispielsweise bei Kataraktoperationen ist eine präzise und aussagekräfte Analyse des Hornhautendothels angebracht. Auch zur prophylaktischen Begleitung von Patienten im Hinblick von kontaktlinseninduzierter Hornhautendothelschädigung ist eine Analyse des Hornhautendothels wünschenswert.

Aufgabe der Erfindung ist es, eine Vorrichtung zur berührungslosen Untersuchung eines Auges, insbesondere von Bestandteilen der Hornhaut zu schaffen, welche möglichst einfach und patientenfreundlich einsetzbar ist.

Diese Aufgabe wird durch die Merkmale des Patentanspruches 1 gelöst.

Bei der Erfindung ist eine mit einer Beleuchtungseinrichtung, insbesondere Spaltlampe mechanisch gekoppelten Bildaufnahmeeinrichtung vorgesehen, mit welcher von der Beleuchtungseinrichtung beleuchtete Bestandteile der Hornhaut, insbesondere Zellen des Hornhautendothels erfasst und mit einer an die Bildaufnahmeeinrichtung angeschlossenen Bildwiedergabeeinrichtung sichtbar dargestellt werden.

Die vorzugsweise als Zusatzgerät ausgebildete Bildaufnahmeeinrichtung arbeitet vollständig berührungslos und benötigt keinerlei Sonden, die mit der Augenoberfläche in Berührung gebracht werden müssen. Infektionsrisiken sowie physische Verletzungsgefahren während der Untersuchung sind somit ausgeschlossen. Eine Lokalanästhesie entfällt vollständig.

Für die Untersuchung können die Beleuchtungseinrichtung mit fest eingestellter Richtung des Beleuchtungsstrahl (Spaltlampenstrahl) und die Bildaufnahmeeinrichtung mit fest eingestellter Blickrichtung bzw. Bildaufnahmeeinrichtung gemeinsam an einem Basiskörper bewegt werden. Die Bildaufnahmeeinrichtung beinhaltet vorzugsweise eine Videokamera mit einem Kameraobjektiv. An die Videokamera sind eine Bildauswerteeinrichtung sowie die Bildwiedergabeeinrichtung angeschlossen.

Die Bildauswerteeinrichtung kann eine Bildselektionseinrichtung aufweisen, welche beim Bewegen der Beleuchtungseinrichtung und der Bildaufnahmeeinrichtung aufeinanderfolgend aufgenommene Videobilder der Hornhautendothelzellen nach ihrer Bildqualität sortiert, wobei mehr als drei Videobilder ausgewertet werden. Zur Speicherung der Videobilder mit guter Bildqualität, insbesondere mit scharf abgebildeten Hornhautendothelzellen kann die Bildauswerteeinrichtung einen Speicher aufweisen. Mit Hilfe eines Rechners kann die Bildauswerteeinrichtung die Zelldichte innerhalb einer bestimmten Bildbereiches des Videobildes ermitteln. Die geometrische Gestalt des Bildbereiches, innerhalb welchem die Analyse insbesondere die Zelldichte zu ermitteln ist, kann von der Bedienungsperson festgelegt werden.

Die Zelldichteverteilung von Zellflächen und Zellformen können innerhalb weniger Sekunden zur Beurteilung und gegebenenfalls zur Nachbearbeitung auf dem Bildwiedergabegerät angezeigt werden. Die standardisierte Arbeitsweise des Systems gewährleistet jederzeit reproduzierbare Ergebnisse. Neben den Videobildaufnahmen können die Analyseergebnisse sowohl zahlenmäßig als auch in graphischer Form dargestellt werden. Die entsprechenden Daten und Videobilder können abgespeichert und jederzeit nachträglich überprüft werden. Auch der Vergleich mit zurückliegenden Untersuchungen ist somit möglich.

Mit der Vorrichtung können auch Bilder der mit der Spaltlampe erzeugten beleuchteten Schnitte der Hornhaut und der auf der Hornhautoberfläche befindlichen Tränenflüssigkeit erzeugt werden. Durch rechnergestützte Auswertung dieser Videobilder lässt sich die Hornhautdicke und/oder die Dicke der Tränenflüssigkeit bzw. des Tränenfilms auf der Hornhautoberfläche bestimmen. Ferner lässt sich die Geschwindigkeit des Tränenfilms durch die aufgenommene Bildfolge bestimmen. Aus der Geschwindigkeit des Tränenfilms sind Rückschlüsse auf dessen Viskosität möglich. Ferner lassen sich aus den Tränenfilmbildern Abrisse des Tränenfilms ermitteln.

Die Erfindung betrifft ferner eine Vorrichtung zur Positionierung eines Objektivs der Bildaufnahmeeinrichtung, mit welcher von der Beleuchtungseinrichtung der Spaltlampe beleuchtete Augenbestandteile, insbesondere Zellen eines Hornhautendothels eines Auges zu erfassen sind.

Dabei wird auf eine einfache Weise eine exakte Ausrichtung und Positionierung des Objektivs der Bildaufnahmeeinrichtung gegenüber der Beleuchtungseinrichtung, insbesondere gegenüber der Spaltlampe erreicht.

Hierzu kann an der Bildaufnahmeeinrichtung, beispielsweise mit Hilfe einer Halteplatte, eine Befestigungseinrichtung vorgesehen sein, welche in eine zur Schwenkachse der Beleuchtungseinrichtung ausgerichteten Bohrung vorzugsweise am Träger der Spaltlampe für ein Verschwenken der Bildaufnahmeeinrichtung um die Schwenkachse eingesetzt werden kann. Auf diese Weise erreicht man, dass die Bildaufnahmeeinrichtung und das Objektiv der Bildaufnahmeeinrichtung um eine gemeinsame Schwenkachse mit der Beleuchtungseinrichtung, insbesondere der Spaltlampe verschwenkt werden können. Hierzu kann die Befestigungseinrichtung eine in die Bohrung am Spaltlampenhalter ragende spreizbare Hülse aufweisen. Mit Hilfe einer durch den Hohlraum der Hülse ragenden Schraubenverbindung kann die Hülse radial nach außen gespreizt werden, so dass das Objektiv der Bildaufnahmeeinrichtung und die Bildaufnahmeeinrichtung in einem bestimmen Winkel zur Beleuchtungseinrichtung des Spaltlampengerätes fixiert werden. Beim radialen Nachaußenspreizen der Hülse wird zwischen der Hülse und der Innenwand der Bohrung ein formschlüssiger Reibschluss gebildet. Bei gelöster oder gelockerter Schraubenverbindung kann die Beleuchtungseinrichtung und die Bildaufnahmeeinrichtung um die gemeinsame Schwenkachse in neue Winkelpositionen verschwenkt werden.

Zur Fixierung der spreizbaren Hülse wird die axiale Vorspannung, welche mit der durch die Hülse ragenden Schraubenverbindung auf die beiden Teile der Schraubenverbindung ausgeübt wird, zur Außendurchmesservergrößerung der Hülse und damit zum formschlüssigen Reibschluss mit der Bohrungsinnenwand ausgenützt. Hierzu kann eine der Schraubenverbindungsteile vorzugsweise eine Mutter, in welche das Gewinde eines Schraubenschaftes eingreift, einen Spreizkonus aufweisen, welcher in einen entsprechend geformten Spreizkonus an der Hülse eingesetzt ist. Aufgrund der von der Schraubenverbindung vermittelten axialen Vorspannung wird dann die Hülse radial nach außen gespreizt. Vorzugsweise sind die Hülse und die Mutter verdrehsicher zueinander in der Bohrung angeordnet.

Hierdurch wird eine Befestigungseinrichtung geschaffen, mit welcher die optische Achse bzw. Blickrichtung des Objektivs der Bildaufnahmeeinrichtung und die Richtung des Beleuchtungsstrahls der Beleuchtungseinrichtung drehfest einen bestimmten Winkel zueinander bei der gemeinsamen Bewegung während der Augenuntersuchung gehalten werden. Diese Befestigungseinrichtung kann auch mit anderen konstruktiven Bauteilen verwirklicht werden. Hierzu kann beispielsweise in die Bohrung am Spaltlampengerät ein an der Bildaufnahmeeinrichtung befestigter Stift verdrehsicher eingesteckt werden. Hierzu können die Bohrung und der Stift von einem kreisrunden Querschnitt abweichende Querschnittsformen aufweisen, sodass die Verdrehsicherung gewährleistet wird. Ferner kann der Stift mit einer mechanischen Verdrehsicherung fest verbunden sein, welche mit der die Bohrung umfassenden Außenkontur an der Spaltlampe, beispielsweise am Schwenkfuß oder Halter der Spaltlampe verdrehsicher in Eingriff kommt.

Durch die beschriebenen Befestigungseinrichtungen kann die Bildaufnahmeeinrichtung bei Bedarf mit einem herkömmlichen Spaltlampengerät verbunden werden. Es ist jedoch auch möglich, die Spaltlampe und die Bildaufnahmeeinrichtung in einer Baueinheit vorzusehen, in welcher sie verdrehsicher in einer bestimmten Winkelstellung zueinander gemeinsam bei der Augenuntersuchung an einem Basiskörper bewegt werden können.

Das Objektiv kann einen Objektivtubus aufweisen, in welchem das Objektivlinsensystem angeordnet ist. Durch Verdrehen des Objektivtubus gegenüber einem an der Bildaufnahmeeinrichtung vorgesehenen Halter kann das Objektivlinsensystem in Richtung seiner optischen Achse verstellt werden. Auf diese Weise lässt sich eine Scharfstellung des abzubildenden Augenbestandteils, insbesondere der vergrößert abzubildenden Zellen des Hornhautendothels für die Bilderfassung, beispielsweise mit der Hilfe einer Videokamera erreichen.

Anhand der Figuren wird an Ausführungsbeispielen die Erfindung noch näher erläutert.

Es zeigt
- Fig. 1: ein Ausführungsbeispiel der Erfindung;
- Fig. 2: eine Draufsicht auf Bestandteile des Ausführungsbeispiels der Fig. 1;
- Fig. 3: einen Ausschnitt eines Videobildes von Hornhautendothelzellen, welches mit dem Ausführungsbeispiel erzeugt wird;
- Fig. 4: eine Justage-Einrichtung für das Ausführungsbeispiel;
- Fig. 5: ein Blockschaltbild, in welchem wesentliche Funktionselemente des Ausführungsbeispiels dargestellt sind;

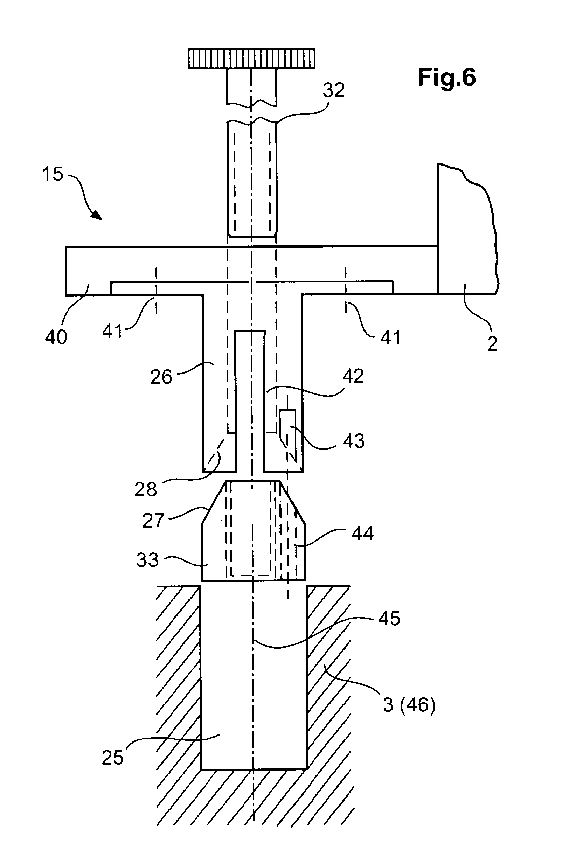

- Fig. 6: ein Ausführungsbeispiel für eine Positioniervorrichtung, mit welchem die Beleuchtungseinrichtung am Spaltlampengerät angeordnet wird;

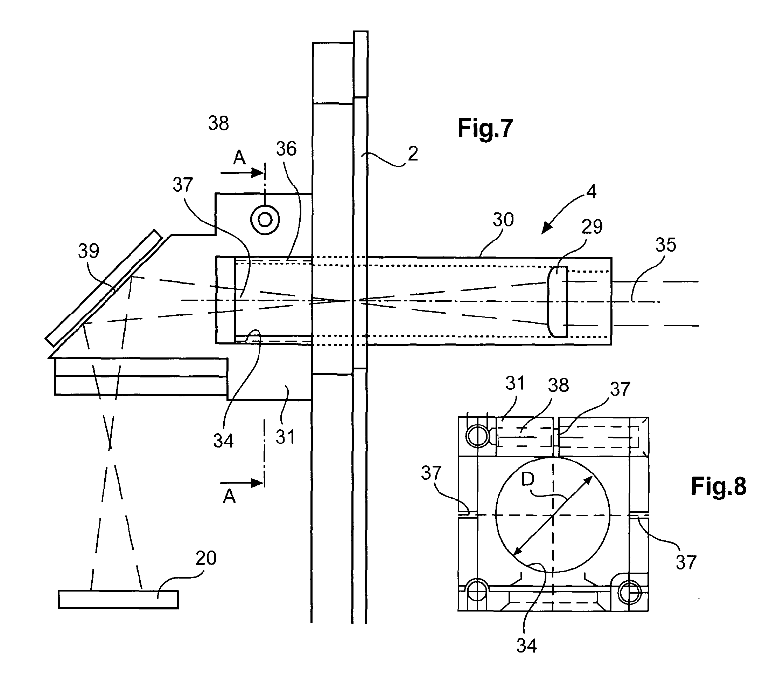

- Fig. 7: schematisch die Anordnung eines Objektivs, welches in der Bildaufnahmeeinrichtung beim Ausführungsbeispiel vorgesehen ist;
- Fig. 8: eine schnittbildliche Darstellung entlang einer Schnittlinie A-A in Fig. 7 durch einen Objekthalter;
- Fig. 9: eine weitere Ausführungsform für die Positioniervorrichtung in perspektivischer Darstellung;
- Fig. 10: noch eine weitere Ausführungsform für die Positioniervorrichtung in perspektivischer Darstellung;
- Fig. 11: eine bei den weiteren Ausführungsbeispielen der Figuren 10 und 11 verwendbare Befestigungseinrichtung.

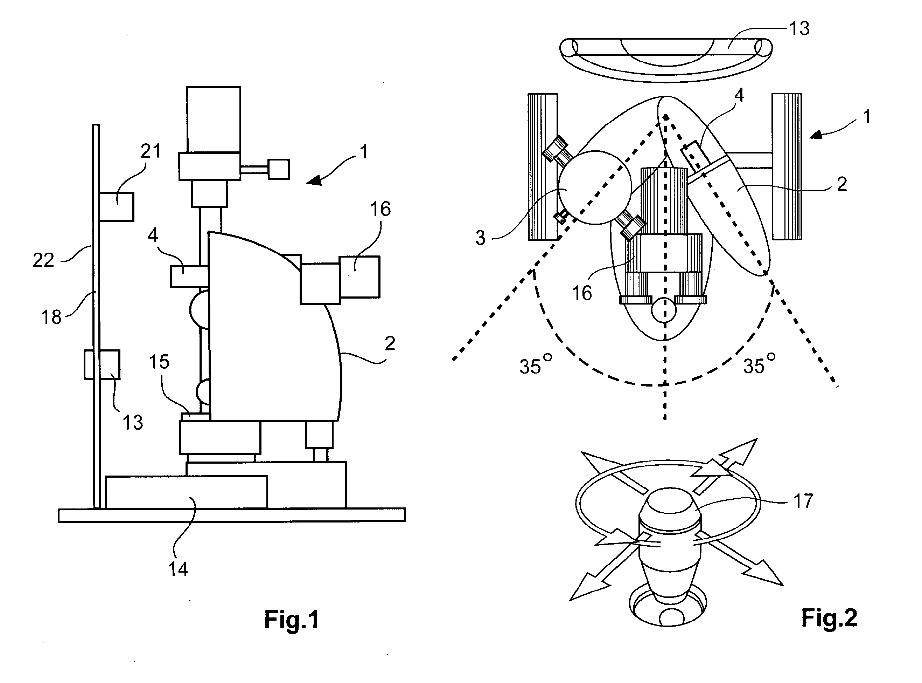

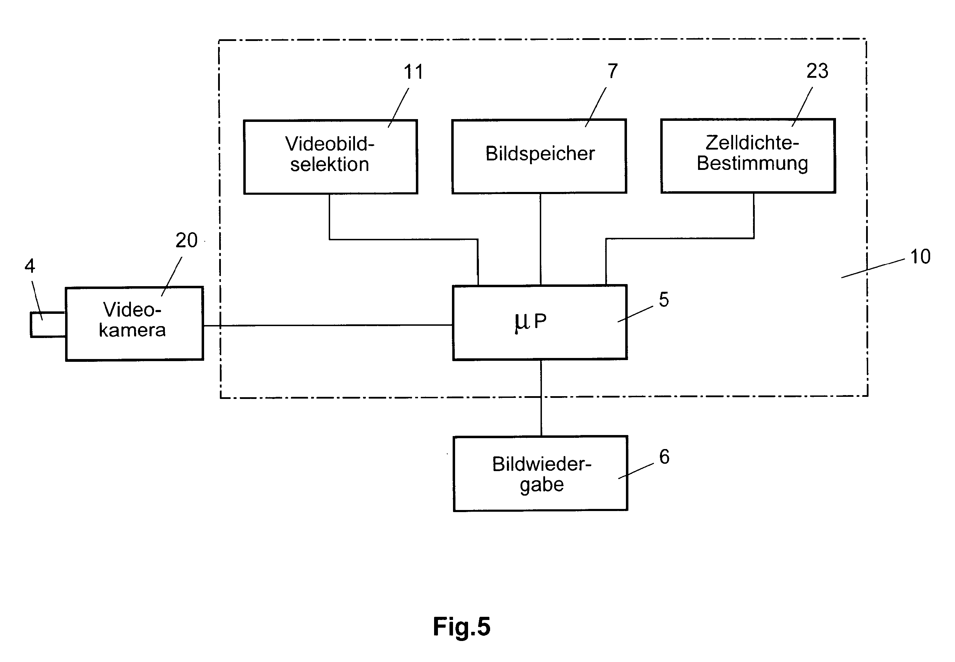

Das in den Fig. 1 und 2 dargestellte Ausführungsbeispiele beinhaltet eine Spaltlampe 1, welches eine herkömmliche Ausführungsform aufweisen kann sowie eine Bildaufnahmeeinrichtung 2. Die Bildaufnahmeeinrichtung 2 und das Spaltlampengerät 1 sind mechanisch so gekoppelt, dass mit Hilfe einer Beleuchtungseinrichtung 3 der Spaltlampe 1 beleuchtete Hornhautendothelzellen erfassbar sind. Mit Hilfe einer an die Bildaufnahmeeinrichtung 2 angeschlossenen Bildwiedergabeeinrichtung 6 (Fig. 5) können die Hornhautendothelzellen sichtbar dargestellt werden. Die Bildaufnahmeeinrichtung 2 weist hierzu in einem Gehäuse eine Videokamera 20 mit einstellbarem Kameraobjektiv 4 auf. Als Bildwiedergabeeinrichtung 6 eignet sich ein Bildschirm eines Laptops oder eines PC-Gerätes oder auch ein geeigneter Monitor. Die Beleuchtungseinrichtung 3 der Spaltlampe erzeugt ein schmales Lichtbüschel, welches in bekannter Weise einen optischen Schnitt durch das transparente Augengewebe insbesondere der Hornhaut des Auges herstellt. Beim dargestellten Ausführungsbeispiel kann das von der Beleuchtungseinrichtung 3 erzeugte Lichtbüschel beispielsweise eine Spaltbreite von ca. 1 mm und eine Spalthöhe von ca. 5 mm aufweisen.

Die Bildaufnahmeeinrichtung 2, welche als Zusatzgerät zur Spaltlampe 1 ausgebildet ist, kann mit einer Befestigungseinrichtung 15, welche bis zum Anschlag in eine Zentralbohrung des Spaltlampengerätes eingesteckt und arretiert wird, mechanisch mit dem Spaltlampengerät 1 gekoppelt werden.

Die mechanische Anbindung an das Spaltlampengerät 1 ist vorzugsweise derart, dass die Beleuchtungseinrichtung 3 des Spaltlampengerätes 1 mit fest eingestellter Richtung des Beleuchtungsstrahls, welcher von dem schmalen Lichtbüschel der Spaltlampe gebildet wird, und die Bildaufnahmeeinrichtung 2 mit fest eingestellter Blickrichtung bzw. Bildaufnahmerichtung gemeinsam an einem Basiskörper 14 des Spaltlampengerätes 1 bewegt werden können.

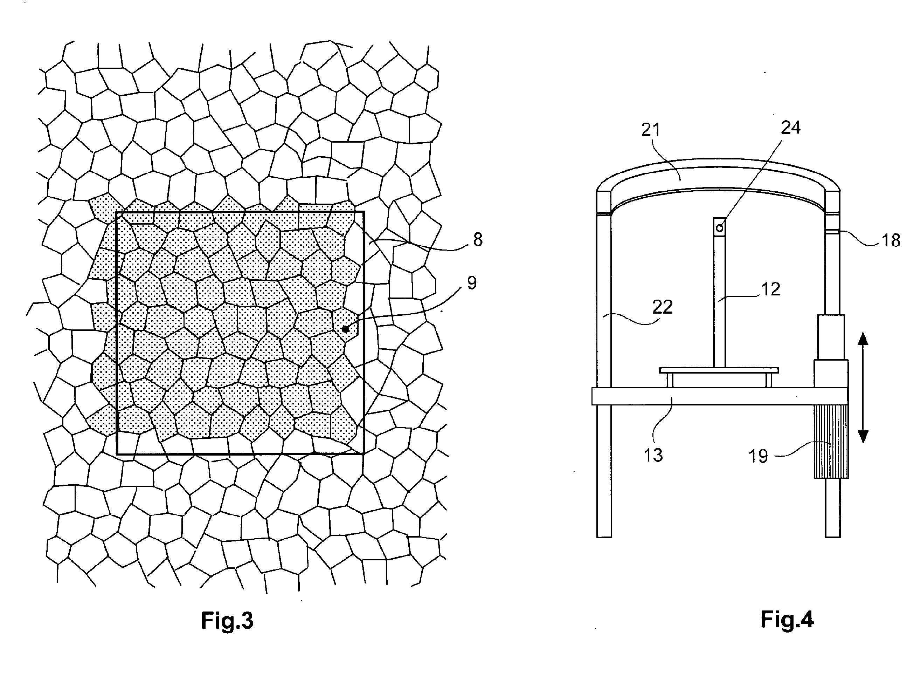

Hierzu kann vorzugsweise vorab eine Justage der Vorrichtung durchgeführt werden. Dabei wird auf eine Kinnstütze 13 ein Justagehilfsgerät 12 gestellt (Fig. 4). Das stabförmige Justagehilfsgerät besitzt an seinem oberen Ende ein Netz 24 oder ein Bild eines Netzes. Es kann auch eine andere Form, welche angenähert dem Zellmuster der aufzunehmenden Hornhautendothelzellen entspricht, am oberen Ende des stabförmigen Justagehilfsgerätes 12 vorgesehen sein. Wie in Fig. 2 dargestellt, wird die Strahlrichtung der Beleuchtungseinrichtung 3 gegenüber der Achse eines binokularen Mikroskops 16 an der Spaltlampe 1 in einem Winkel von etwa 35° eingestellt. Desgleichen wird die Blickrichtung bzw. die optische Achse des Kameraobjektivs 4 gegenüber der Achse des Mikroskops 16 ebenfalls in einem Winkel von ca. 35° eingestellt. Die Beleuchtungsrichtung und die optische Achse des Kameraobjektivs 4 bzw. die Blickrichtung der Aufnahmeeinrichtung 2 schließen somit einen Winkel von etwa 70° ein (Fig. 2). Der Spalt für das Lichtbüschel der Spaltlampe wird möglichst schmal eingestellt. Mittels einer Einstelleinrichtung 17, beispielsweise einem Joy-Stick, welcher in einer Ebene die gemeinsame Bewegung von Beleuchtungseinrichtung 3 und Bildaufnahmeeinrichtung 2 in allen Bewegungsrichtungen steuern kann, wird die Beleuchtungseinrichtung 3 und die Bildaufnahmeeinrichtung 2 so bewegt, dass das Lichtbüschel der Spaltlampe etwa mittig auf dem Netz 24 abgebildet wird. Bei fixierter Anordnung wird das verstellbare Kameraobjektiv 4 so eingestellt, dass das Videobild des Netzes auf der Bildwiedergabeeinrichtung 6, beispielsweise dem Monitorbildschirm oder dergleichen mittig und mit möglichst scharfen Umrisslinien erscheint.

Damit das Netz 24 in Höhe der optischen Achse des Kameraobjektivs liegt, kann die Kinnstütze 13 mit Hilfe einer Stellschraube oder einem anderen gleichwirkenden Stellmittel verstellt werden, sodass das Netz 24 in die Höhe einer an einem Rahmen 22 der Kinnstütze 13 vorgesehenen Markierung 18 gelangt.

Bei der Untersuchung eines Patientenauges liegt das Kinn des Patienten auf der Kinnstütze 13 auf und die Stirn des Patienten liegt fest gegen eine Kopfstütze 21 am Rahmen 22 an. Mit Hilfe der Stellschraube 19 wird das zu untersuchende Patientenauge in die Höhe der Markierung 18 und damit in die Höhe der optischen Achse des Kameraobjektivs 4 gebracht.

Beim Aufnahmemodus wird während der Untersuchung der optische Schnitt, den das Lichtbüschel der Spaltlampe an der Hornhaut des Patienten erzeugt, scharf und mittig auf der Hornhaut eingestellt. Die Position des optischen Schnittes kann je nach Blickrichtung des Patienten leicht varüeren. Gegebenenfalls kann bei Erreichen der mittigen Position von der Bildauswerteeinrichtung ein Signal, insbesondere optisches Signal abgegeben werden. Mit Hilfe der Einstelleinrichtung 17 kann eine Feineinstellung der gemeinsam bewegten Anordnung bestehend aus der Bildaufnahmeeinrichtung 2 und der Beleuchtungseinrichtung 3 durchgeführt werden, bis die Hornhautendothelzellen mittig und scharf auf der Wiedergabeeinrichtung 6 abgebildet werden. Während dieser gesteuerten Bewegung der Bildaufnahmeeinrichtung 2 und der Beleuchtungseinrichtung 3 werden von der Videokamera 20, welche in der Bildaufnahmeeinrichtung 2 vorgesehen ist, aufeinanderfolgende Endothelbilder gefertigt, die am Bildwiedergabegerät 6 sichtbar gemacht werden. Es werden mehr als drei Videobilder ausgewertet, wobei beispielsweise bis zu 50 aufeinanderfolgende Videobilder gemacht und ausgewertet werden können. Bilder mit guter Wiedergabequalität werden in einem Bildspeicher 7 einer Bildauswerteeinrichtung 10 (Fig. 5) zwischengespeichert. Die Auswahl der Bilder mit guter Wiedergabequalität kann mit Hilfe einer Bildselektionseinrichtung 11 der Bilderauswerteeinrichtung 10 erfolgen. Die Untersuchung am Patientenauge kann beendet werden, wenn auswertbare Endothelbilder vorliegen und am Bildwiedergabegerät 6 sichtbar waren. Aus den im Bildspeicher 7 zwischengespeicherten Videobildern kann ein geeignetes Videobild ausgewählt und am Wiedergabegerät 6 sichtbar gemacht werden. In der Fig. 3 ist ein Bildausschnitt der am Bildwiedergabegerät 6 gezeigten Hornhautendothelzellen schematisch dargestellt.

Für die Auswertung kann ein bestimmter Bildbereich 8, welcher durch Umgrenzungslinien am Wiedergabegerät 6 festgelegt wird, für eine Zelldichtenanalyse ausgewählt werden, wie es in Fig. 3 dargestellt ist. Die im Bildbereich 8 liegenden Endothelzellen können mit einer Markierung 9 versehen werden. Es kann sich um eine flächige oder auch punktförmige Farbmarkierung der einzelnen innerhalb des bestimmten Bildbereichs 8 abgebildeten liegenden Zellen handeln. Gegebenenfalls kann mit Hilfe von Maus-Klick eine Korrektur der Farbmarkierung und auch eine Korrektur der jeweiligen Positionen der Umrandungslinien, welche den bestimmten Bildbereich 8 umfassen, durchgeführt werden. Der bestimmte Bildbereich 8 kann eine rechteckige, quadratische oder eine andere geeignete geometrische Gestalt aufweisen. Durch rechnergestützte Erfassung, beispielsweise Zählung der in dem bestimmten Bildbereich 8 vorhandenen vorzugsweise markierten Zellen lässt sich die Zelldichte in der Auswerteeinrichtung 10 mit Hilfe eines Rechners 5 der Auswerteeinrichtung 10 unter Berücksichtigung des Flächeninhalts des bestimmten Bildbereiches 8 bestimmen (Zelldichtebestimmung 23 in Fig. 5).

Hierzu ist die Auswerteeinrichtung 10, wie aus Fig. 5 zu ersehen ist, an die Videokamera 20, welche in der Bildaufnahmeeinrichtung 2 vorgesehen ist, angeschlossen und wertet die von der Videokamera gelieferten digitalisierten elektrischen Videosignale für die Wiedergabe am Bildwiedergabegerät 6 aus.

Die Bildauswerteeinrichtung 10 kann im Gehäuse der Bildaufnahmeeinrichtung 2 untergebracht sein. Es ist jedoch auch möglich, die Funktionen der Auswerteeinrichtung 10 auf einem transportablen Speicher, beispielsweise einer Diskette als Software zu speichern und in den dem Wiedergabegerät 6 zugeordneten Computer bei der jeweiligen Untersuchung zu installieren. Durch diese Analysesoftware kann eine Steuerung des jeweiligen Live-Videobildes des Hornhautendothels, eines zentralen Messpunktes des Hornhautendothels, einer automatischen Bildselektion der Darstellung der Zelldichte der Zellflächen und der Zellmorphologie erfolgen. Ferner erreicht man hierdurch eine einfache und schnelle Analyse des Hornhautendothels.

Damit Bilder mit guter Bildwiedergabequalität erreicht werden, ist es von Vorteil, wenn die Beleuchtungseinrichtung der Spaltlampe 1 und das Objektiv 4 der Bildaufnahmeeinrichtung 2 exakt zueinander eingestellt sind. Hierzu dient eine Positioniereinrichtung, mit welcher gewährleistet ist, dass die Bildaufnahmeeinrichtung 2 und ihr Objektiv 4 sowie die Beleuchtungseinrichtung 3 um eine gemeinsame Schwenkachse 45 geschwenkt werden. Beim in der Fig. 3 dargestellten Ausführungsbeispiele sind Einzelheiten der Befestigungseinrichtung 15 dargestellt, mit welcher diese Anforderung erfüllt wird. Hierzu ist am Spaltlampengerät, insbesondere am Schwenkfuß der Beleuchtungseinrichtung 3 um die Schwenkachse 45 eine Bohrung 25 mit kreisförmigem Durchmesser vorgesehen. In diese Bohrung 25 wird eine Mutter 33 eingesetzt, die an ihrer nach oben weisenden Fläche konisch ausgebildet ist, wodurch ein Spreizkonus 27 mit nach oben gerichteter konischer Ringfläche geschaffen wird. Auf den Spreizkonus 27 wird eine Hülse 26 aufgesetzt. An der Unterseite der Hülse 26 befindet sich ebenfalls ein Spreizkonus 28 mit einer im wesentlichen ringförmigen Konusfläche, die an den Spreizkonus 27 angepasst ist. Die Hülse weist parallel zu ihrer Achse verlaufende Schlitze 42 auf, sodass eine radial nach außen gerichtete Verformung des Hülsenmantels erfolgen kann. Die Hülse befindet sich ebenfalls in der Bohrung 25, wenn sie auf den Spreizkonus der Mutter 33 aufgesetzt ist.

Die Hülse 26 weist einen Flansch auf, welcher mit Schraubverbindungen 41 an der Unterseite einer Halteplatte 40 befestigt ist. Die Halteplatte 40 ist am Gehäuse der Bildaufnahmeeinrichtung 2 befestigt.

Durch eine Bohrung in der Halteplatte 40 und den Hohlraum in der Hülse 26 kann eine Schraube 33 hindurchgesteckt werden und mit dem Gewinde am Schraubenschaft mit der Mutter 33 verschraubt werden. Beim Festziehen der so geschaffenen Schraubenverbindung wird eine axiale Vorspannung erzeugt, durch welche die beiden Spreizkonen 27 und 28 gegeneinander gedrückt werden. Die Mutter 33 und die Hülse 26 können beim Festziehen der Schraubenverbindung dadurch verdrehsicher gegeneinander gehalten werden, dass in Bohrungen 43 und 44 in der Hülse 26 und der Mutter 33 ein Stift angeordnet wird. Aufgrund der Spreizbarkeit der Hülse 26 wird die Mantelfläche der Hülse 26 gegen die Innenwand der Bohrung 25 gedrückt, sodass durch Reibschluss eine Fixierung sowohl in axialer Richtung als auch in Drehrichtung erreicht wird. Auf diese Weise wird mit einfachen Mitteln eine Positionierung der Bildaufnahmeeinrichtung 2 am Spaltlampengerät 1 und insbesondere an der Beleuchtungseinrichtung 3 erreicht.

Zur erleichterten Betätigung der Schraubenverbindung kann die Schraube 32 als Rändelschraube ausgebildet sein, welche im gelösten Zustand, beispielsweise durch einen Sprengring gegenüber der Halteplatte 40 gesichert ist, sodass ein Herausfallen der Schraube 32 auch bei gelöster oder gelockerter Schraubenverbindung vermieden wird. Bei gelöster oder gelockerter Schraubenverbindung kann die Bildaufnahmeeinrichtung 2 um die Schwenkachse 45, welche eine gemeinsame Schwenkachse mit der Beleuchtungseinrichtung 3 bilden kann, geschwenkt werden und in eine gewünschte Winkelposition gebracht werden. Durch die oben erläuterte Fixierung wird diese gewünschte Winkelpositionierung bei der Augenuntersuchung beibehalten.

Hierdurch wird erreicht, dass die optische Achse 35 des Objektivs 4 auf den vom Lichtbüschel der Spaltlampen erzeugten Schnitt an der Hornhaut des Patienten gerichtet ist. Das Objektiv 4 weist einen Objektivtubus 30 auf, welcher mit einem Außengewinde 36 in ein Innengewinde 34 eines Objektivhalters 31 eingeschraubt wird. Der Objektivhalter 31 ist an der Innenseite des Gehäuses der Beleuchtungseinrichtung 3 befestigt. Der Objektivtubus 30 ragt über die Vorderfläche des Gehäuses der Bildaufnahmeeinrichtung 2 hinaus, wie es aus den Figuren zu ersehen ist. Im Objektivtubus 30 ist ein Objektivlinsensystem 29 angeordnet, welches beim Verdrehen des Objektivtubusses 30 axial in Richtung der optischen Objektivachse 35 verstellt wird.

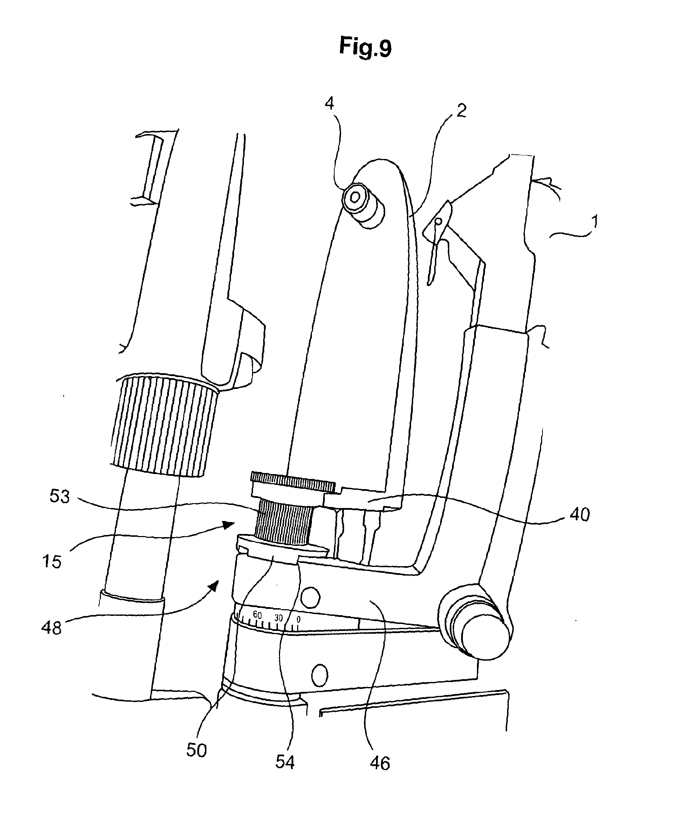

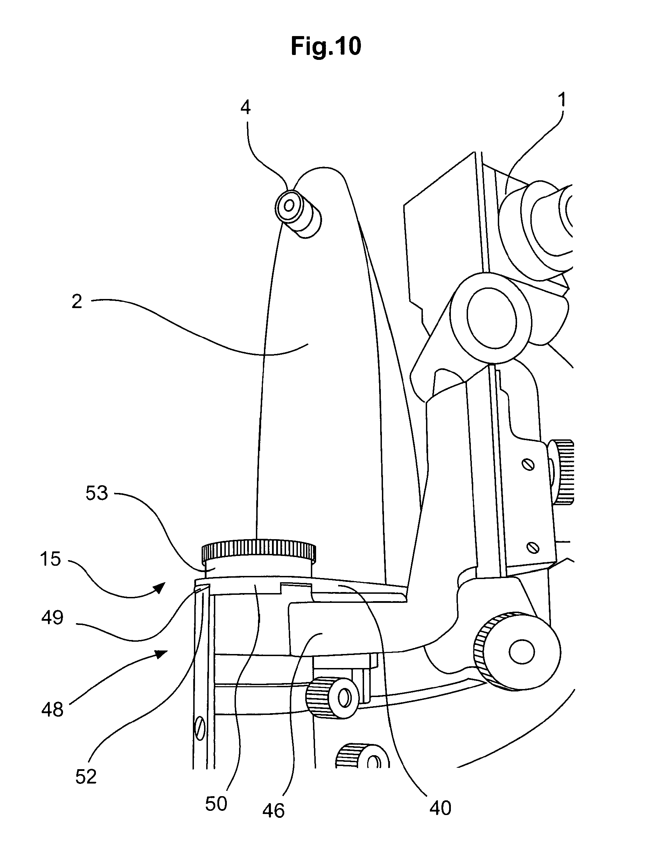

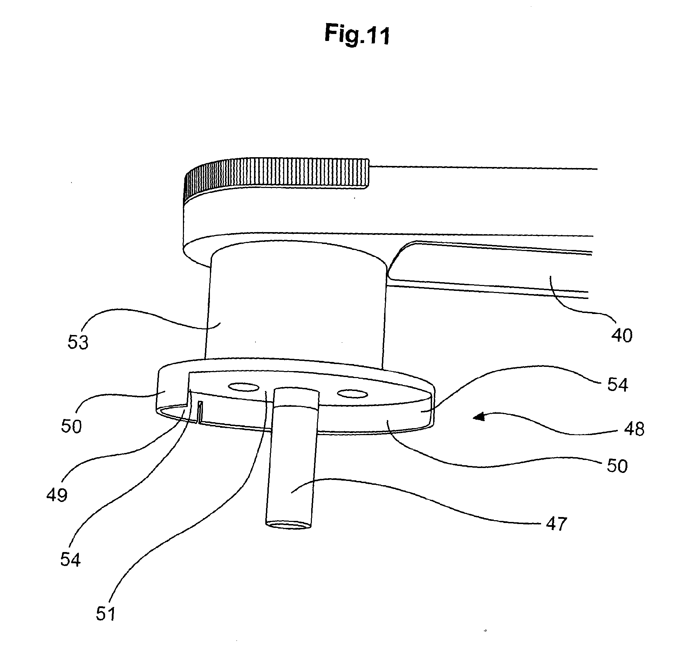

Die Gängigkeit der Verdrehbewegung und damit der axialen Verschiebbarkeit des Objektivs 4 kann eingestellt werden. Hierzu ist bzw. sind dann am Objektivhalter 31 ein oder mehrere Schlitze 37, die sich parallel zur optischen Objektivachse 35 erstrecken, vorgesehen. Mit Hilfe einer senkrecht dazu sich erstreckenden Klemmschraube 38 lässt sich der Durchmesser D (Fig. 5) des Innengewindes 34 am Objektivhalter 31 verändern. Diese Veränderung kann zwischen Leichtgängigkeit bis zum Klemmsitz des Objektivs 4 im Objektivhalter 31 erfolgen. Das Material des Objektivhalters 31 ist hierzu entsprechend verformbar ausgebildet. Auf diese Weise kann die eingestellte Brennweite und gegebenenfalls Vergrößerung des zu erfassenden Bildes im optischen Schnitt des Lichtbüschels der Spaltlampe fixiert werden. Der Strahlengang des Objektivs 4 wird in der Bildaufnahmeeinrichtung 2 über einen Umlenkspiegel 39, welcher gehäusefest angeordnet ist, auf die Videokamera und hier insbesondere auf deren helligkeitsempfindliche Fläche gerichtet. Die Videokamera 20 kann beispielsweise als CCD-Kamera ausgebildet sein. Wie schon erläutert, ist an die Videokamera 20 eine Bildwiedergabeeinrichtung, welche nicht näher dargestellt ist, angeschlossen. In den Figuren 9 bis 11 sind weitere Ausführungsbeispiele zur drehfesten Verbindung der Bildaufnahmeeinrichtung 2 mit der Spaltlampe 1 dargestellt. Die bei diesen Ausführungsbeispielen vorgesehene Befestigungseinrichtung 15 weist einen an der Bildaufnahmeeinrichtung 2 befestigten Stift 47 (Figur 11) auf, welcher in die am Spaltlampengerät vorgesehene Bohrung 25 eingesteckt werden kann. Bei den Ausführungsbeispielen der Figuren 9 und 10 ist der Stift 47 in die Bohrung 25 eingesteckt. Die Bohrung 25 befindet sich am Ende eines Halters 46 für die Spaltlampe 1. Bei diesem Halter 46 handelt es sich bei den Ausführungsbeispielen um den Schwenkfuß der Spaltlampe 1. Der Stift 47 ist am Ende der Halteplatte 40 befestigt und über diese Halteplatte 40 fest mit der Bildaufnahmeeinrichtung 2 verbunden. Die Befestigung des Stiftes 47 an der Unterseite der Halteplatte 40 kann über einen Abstandhalter 53 erfolgen, dessen Höhe je nach Ausbildung der am Halter 46 ausgebildeten Halterlagerung bemessen ist. Beim Ausführungsbeispiel der Figur 9 ist die Höhe des Abstandhalters 53 größer bemessen als beim Ausführungsbeispiel der Figur 10.

An der Unterseite des Abstandhalters 52 ist eine Platte 51 befestigt, welche an ihrem Umfangsrand einen nach unten ragenden Kragen 50 aufweist. Die Unterseite der Platte 51 bildet eine Auflagefläche, welche auf die oben liegende Fläche der Drehlagerung für den Halter 46 der Spaltlampe 1 auflegbar ist. Der nach unten ragende Kragen 50 umfasst dabei bei den Ausführungsbeispielen teilweise die Außenkontur der Drehlagerung des Halters 46. Durch die besondere Ausgestaltung des Kragens 50 in Zusammenwirkung mit der Außenkontur der Drehlagerung des Halters 46 wird eine Verdrehsicherung 48 geschaffen, durch welche bei der Befestigungseinrichtung 15 die drehfeste Verbindung der Bildaufnahmeeinrichtung mit der Spaltlampe 1 erreicht wird. Die optische Achse des Kameraobjektivs 4 und die Richtung des Beleuchtungsstrahles der Spaltlampe 1 sind damit drehfest miteinander verbunden.

Beim Ausführungsbeispiel der Figur 9 erfolgt die drehfeste Verbindung dadurch, dass der Halter 46 sich durch eine der Breite des Halters 46 angepasste Ausnehmung im Kragen 50 ragt. Diese Ausnehmung im Kragen 50 wird begrenzt durch Anschlagflächen 54, welche seitlich am Halter 46 anliegen. Die übrigen Teile des Kragens 50 ragen nach unten und liegen an der Außenkontur der Drehlagerung des Halters 46 an, wie dies aus der Figur 9 zu ersehen ist.

Beim Ausführungsbeispiel der Figur 10 ist an der Drehlagerung des Halters 46 ein Vorsprung 52 vorgesehen, welcher in eine Ausnehmung 49 des Kragens 50 ragt. Hierdurch wird die verdrehsichere Positionierung der Bildaufnahmeeinrichtung 2 gegenüber der Spaltlampe erreicht.

Bei den Ausführungsbeispielen der Figuren 9 bis 11 erzielt man eine einfache Positionierung der Bildaufnahmeeinrichtung 2 im Bereich der Drehlagerung der Spaltlampe 1 an deren Halter 46 bzw. Schwenkfuß. Die Bildaufnahmeeinrichtung 2 kann in einfacher Weise in der Befestigungseinrichtung 15 durch Einstecken des Stiftes 47 in die Bohrung 25 an der Drehlagerung erreicht werden. Auch das Entfernen der Bildaufnahmeeinrichtung 2 aus der Befestigungseinrichtung 15 gestaltet sich durch Herausziehen des Stiftes 47 aus der Bohrung 25 einfach.

Bezugszeichenliste
- 1: Spaltlampe
- 2: Bildaufnahmeeinrichtung
- 3: Beleuchtungseinrichtung
- 4: Kameraobjektiv
- 5: Rechner
- 6: Bildwiedergabegerät
- 7: Bildspeicher
- 8: bestimmter Bildbereich
- 9: Zellenmarkierung
- 10: Bildauswerteeinrichtung
- 11: Bildselektionseinrichtung
- 12: Justagehilfsgerät
- 13: Kinnstütze
- 14: Basiskörper
- 15: Befestigungseinrichtung
- 16: okkulares Mikroskop
- 17: Einstelleinrichtung
- 18: Augenhöhenmarkierung
- 19: Stellschraube
- 20: Videokamera (CCD-Kamera)
- 21: Kopfstütze
- 22: Rahmen
- 23: Zelldichteerfassung
- 24: Netz
- 25: Bohrung
- 26: Hülse
- 27: Spreizkonus
- 28: Spreizkonus
- 29: Linsensystem
- 30: Objektivtubus
- 31: Objektivtubus
- 32: Schraube
- 33: Mutter
- 34: Innengewinde im Objektivhalter
- 35: Objektivachse
- 36: Außengewinde am Objektivhalter
- 37: Schlitze
- 38: Klemmschraube
- 39: Umlenkspiegel
- 40: Halteplatte
- 41: Schraubenverbindung
- 42: Schlitze
- 43: Bohrung
- 44: Bohrung
- 45: Schwenkachse
- 46: Halter
- 47: Stift
- 48: Verdrehsicherung
- 49: Ausnehmung
- 50: Kragen
- 51: Platte
- 52: Vorsprung
- 53: Abstandhalter
- 54: Anschlagflächen

## Patentansprüche

1. Vorrichtung zur berührungslosen Untersuchung eines Auges, enthaltend eine mit einer Spaltlampe (1) mechanisch gekoppelte Bildaufnahmeeinrichtung (2), mit welcher von einer Beleuchtungseinrichtung (3) der Spaltlampe (1) beleuchtete Bestandteile der Hornhaut und/oder ein Tränenfilm auf der Hornhautoberfläche erfassbar sind, und eine an die Bildaufnahmeeinrichtung (1) angeschlossene Bildwiedergabeeinrichtung (6), mit welcher die erfassten Bestandteile der Hornhaut und/oder der Tränenfilm im von der Spaltlampe erzeugten Schnittbild sichtbar darstellbar sind oder ist.

2. Vorrichtung nach Anspruch 1, bei welcher die Beleuchtungseinrichtung (3) der Spaltlampe (1) und die Blickrichtung der Bildaufnahmeeinrichtung (2) fest zueinander eingestellt sind und die Bildaufnahmeeinrichtung (2) mit der Spaltlampe (1) gemeinsam an einem Basiskörper (14) bewegbar sind.

3. Vorrichtung nach Anspruch 2, bei welcher die Beleuchtungseinrichtung (3) und die Bildaufnahmeeinrichtung (2) um eine gemeinsame Achse (45) schwenkbar sind.

4. Vorrichtung nach einem der Ansprüche 1 bis 3, bei welcher die Bildaufnahmeeinrichtung (2) eine Videokamera (20) mit einem Kameraobjektiv (4) aufweist, wobei an die Videokamera (20) eine Bildauswerteeinrichtung (10) und das Bildwiedergabegerät (6) angeschlossen sind.

5. Vorrichtung nach einem der Ansprüche 1 bis 4, bei welcher die Bildauswerteeinrichtung (10) eine Bildselektionseinrichtung (11) aufweist, welche beim Bewegen der Beleuchtungseinrichtung (3) und der Bildaufnahmeeinrichtung (2) aufeinanderfolgend aufgenommene Videobilder nach ihrer Bildqualität sortiert.

6. Vorrichtung nach einem der Ansprüche 1 bis 5, bei welcher die Bildauswerteeinrichtung (10) zur Speicherung zumindest der Videobilder mit guter Bildqualität einen Bildspeicher (7) aufweist.

7. Vorrichtung nach einem der Ansprüche 1 bis 6, bei welcher die Bildauswerteeinrichtung (6) einen Rechner (5) aufweist, welcher die Zelldichte von Hornhautendothelzellen und/oder die Hornhautdicke und/oder die Tränenfilmdicke und/oder die Geschwindigkeit des Tränenfilms auf der Hornhautoberfläche, insbesondere innerhalb eines bestimmten Bildbereichs (8) des Videobildes ermittelt.

8. Vorrichtung nach einem der Ansprüche 1 bis 7, bei welcher die den Bildbereich (8) umgebenden Begrenzungslinien auf der Bildwiedergabeeinrichtung (6) sichtbar darstellbar sind.

9. Vorrichtung nach einem der Ansprüche 1 bis 8, bei welcher die in dem bestimmten Bildbereich (8) abgebildeten Hornhautendothelzellen markierbar sind.

10. Vorrichtung nach einem der Ansprüche 1 bis 9, bei welcher ein Justagehilfsgerät (12) an einer Kinnstütze (13) mit einem von der Beleuchtungseinrichtung (3) beleuchteten und in etwa Augenhöhe vorgesehenen Netz (24) positionierbar ist, wobei zur Justage ein scharfes Videobilder mittels Verstellen eines Kameraobjektivs (4) einstellbar ist.

11. Vorrichtung nach einem der Ansprüche 1 bis 10, bei welcher das Kameraobjektiv (4) mit seiner optischen Achse etwa in Höhe des zu untersuchenden Auges anzuordnen ist.

12. Vorrichtung nach einem der Ansprüche 1 bis 11, bei welcher die Kinnstütze (13) höhenverstellbar ist.

13. Vorrichtung nach einem der Ansprüche 1 bis 12, bei welcher mittels einer Befestigungseinrichtung (15) die optische Achse des Objektivs (4) der Bildaufnahmeeinrichtung (2) und die Richtung des Beleuchtungsstrahles der Spaltlampe (1) in einer bestimmten Winkelposition zueinander gehalten sind.

14. Vorrichtung nach einem der Ansprüche 1 bis 13, bei welcher die verdrehsicher mittels der Befestigungseinrichtung (15) miteinander verbundenen Bildaufnahmeeinrichtung (2) und Spaltlampe (1) gemeinsam an einer Basisplatte (14) in allen Bewegungsrichtungen gesteuert bewegbar gelagert sind.

15. Vorrichtung nach einem der Ansprüche 1 bis 14, bei welcher die Bildaufnahmeeinrichtung (2) und die Spaltlampe (1) in der Befestigungseinrichtung (15) durch eine Steckverbindung miteinander verbindbar sind.

16. Vorrichtung nach einem der Ansprüche 1 bis 15, bei welcher die Befestigungseinrichtung (15) eine stiftförmige Steckverbindung (26, 33; 47) aufweist, die in eine Bohrung (25) eines Halters (46) der Spaltlampe (1) steckbar ist.

17. Vorrichtung nach einem der Ansprüche 13 bis 16, bei welcher die Befestigungseinrichtung (15) an der Bildaufnahmeeinrichtung (2) eine spreizbare Hülse (7) aufweist, welche in die zur Schwenkachse (24) der Beleuchtungseinrichtung (3) ausgerichteten Bohrung (25) am Spaltlampengerät (1) für ein Verschwenken der Bildaufnahmeeinrichtung (2) um die Schwenkachse (24) einsetzbar ist und dass durch eine durch den Hohlraum der Hülse (26) ragende Schraubenverbindung (32, 33) die Hülse (26) zur Fixierung des Objektivs der Bildaufnahmeeinrichtung (2) in einem bestimmten Winkel gegenüber der Beleuchtungseinrichtung (3) durch formschlüssigen Reibschluss mit der Innenwand der Bohrung (25) radial nach außen speizbar ist.

18. Vorrichtung nach Anspruch 17, bei welcher wenigstens an einem Schraubenverbindungsteil der Schraubenverbindung (32, 33) ein Spreizkonus (27) vorgesehen ist, welcher in einen entsprechenden Spreizkonus (28) an der Hülse (26) eingreift.

19. Vorrichtung nach einem der Ansprüche 13 bis 16, bei welcher die Befestigungseinrichtung (15) eine mechanische Verdrehsicherung aufweist, welche in die Außenkontur des Halters (46) der Spaltlampe (1) verdrehsicher eingreift.

20. Vorrichtung nach Anspruch 19, bei welcher die Verdrehsicherung eine auf die Drehlagerung des Halters (46) auflegbare Auflagefläche (51) aufweist, welche von einem teilweise umlaufenden Kragen (50) umgeben ist, der mit der Außenkontur der Drehlagerung des Halters (46) verdrehsicher in Eingriff bringbar ist.

21. Vorrichtung nach einem der Ansprüche 1 bis 20, **dadurch gekennzeichnet, dass** das in einem Objektivtubus (30) angeordnete Objektivlinsensystem (29) durch Verdrehen des Objektivtubusses gegenüber einem Gehäuse der Bildaufnahmeeinrichtung (2) in Richtung der optischen Objektivachse (35) verstellbar ist.

22. Vorrichtung nach Anspruch 21, **dadurch gekennzeichnet, dass** die Gängigkeit der Verstellbewegung des Objektivs (4) einstellbar ist.

23. Vorrichtung nach Anspruch 21 oder 22, **dadurch gekennzeichnet, dass** zur axialen Verstellbarkeit des Objektivs (4) der Objektivtubus (30) mit Gewindeeingriff (34, 36) in einem an der Bildaufnahmeeinrichtung (2) vorgesehenen Objektivhalter (31) axial verschiebbar gehalten ist.

24. Vorrichtung nach einem der Ansprüche 21 bis 23, **dadurch gekennzeichnet, dass** der Gewindedurchmesser des am Objektivhalter (31) vorgesehenen Innengewindes (34) veränderbar ist.

25. Vorrichtung nach einem der Ansprüche 21 bis 24, **dadurch gekennzeichnet, dass** der Objektivhalter (31) eine oder mehrere parallel zur optischen Objektivachse (35) verlaufende Schlitze (37) aufweist, dessen oder deren Schlitzbreite durch Veränderung des Gewindedurchmessers am Objektivhalter (31) mittels einer Klemmschraube (38) veränderbar ist.

26. Vorrichtung nach einem der Ansprüche 21 bis 25, **dadurch gekennzeichnet, dass** in der Bildaufnahmeeinrichtung (2) in Strahlengangrichtung nach dem Ojektiv (4) ein Umlenkspiegel (39) angeordnet ist, welcher den Strahlengang durch das Objektiv (4) in Richtung zu einer Videokamera (20) lenkt.

27. Vorrichtung nach Anspruch 26, **dadurch gekennzeichnet, dass** der Umlenkwinkel etwa 90° beträgt
